# EUROPEAN PATENT SPECIFICATION

(11) **EP 2 640 714 B1**
(45) Date of publication and mention of the grant of the patent: **10.09.2014**
(21) Application number: 11779425.5
(22) Date of filing: 08.11.2011
(51) Int. Cl.: C07D 317/34

(54) **PROCESS FOR THE PREPARATION OF 2-OXO-[1,3]DIOXOLANE-4-CARBOXYLIC ACID ESTERS**
VERFAHREN ZUR HERSTELLUNG VON [2-OXO- 1,3] DIOXOLAN-4-CARBONSÄUREESTERN
PROCÉDÉ DE PRÉPARATION D'ESTERS D'ACIDE 2-OXO-[1,3]DIOXOLANE-4-CARBOXYLIQUE

(30) Priority: 16.11.2010 EP 10191334
(43) Date of publication of application: 25.09.2013
(73) Proprietor: BASF SE, 67056 Ludwigshafen (DE)
(72) Inventor: MILLER, Jeremie, Pittsburgh, PA 15217 (US); TELES, Joaquim Henrique, 67165 Waldsee (DE); KLOPSCH, Rainer, 67551 Worms (DE); SEELIG, Bianca, 68169 Mannheim (DE)
(86) International application number: PCT/EP2011/069626
(87) International publication number: WO 2012/065879

(56) References cited:
- ALVARO M ET AL: "CO2 fixation using recoverable chromium salen catalysts: use of ionic liquids as cosolvent or high-surface-area silicates as supports", 15 November 2004 (2004-11-15), JOURNAL OF CATALYSIS, ACADEMIC PRESS, DULUTH, MN, US, PAGE(S) 254 - 258, XP004607482, ISSN: 0021-9517 page 257, table 2
- ALVARO M ET AL: "Polymer-bound aluminium salen complex as reusable catalysts for CO2 insertion into epoxides", TETRAHEDRON, ELSEVIER SCIENCE PUBLISHERS, AMSTERDAM, NL, vol. 61, no. 51, 19 December 2005 (2005-12-19), pages 12131-12139, XP025382868, ISSN: 0040-4020, DOI: 10.1016/J.TET.2005.07.114 [retrieved on 2005-12-19]

## Description

This patent application claims the benefit of pending US provisional patent application Serial Number 61/414001 filed November 16, 2010 incorporated in its entirety herein by reference.

The present invention relates to a process for the preparation of 2-oxo-[1,3] dioxolane-4-carboxylic acid esters of formula I 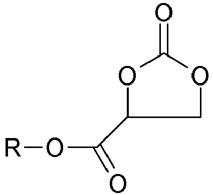 with R being an organic group comprising 1 to 10 carbon atoms, wherein
an oxirane-2-carboxylic acid ester of formula II 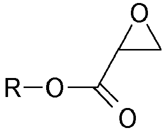 is reacted with carbon dioxide (CO₂) at a pressure of at least 35 bars and a temperature of 60 to 80°C.

Due to their reactivity compounds with a 2-oxo-[1,3] dioxolane group are suitable as starting materials for the preparation of chemical compounds.

EP-A 0001088 discloses 2-oxo-[1,3] dioxolane-4-carboxylic acid esters and their use for the preparation of polymers and/or modification of polymers with reactive groups.

Object of European patent application 10166244.3 (PF 70965, not yet published) are 2-oxo-[1,3] dioxolane-4-carboxylic acid esters of formula I and compounds obtained by reacting 2-oxo-[1,3] dioxolane-4-carboxylic acid esters with polyols.

Alvaro et al, Journal of Catalysis, pp 254-258,2004 and Alvaro et al, Tetrahedron, vol. 61 (51),pp 12131-12139,2005 discloses CO2 insertion into epoxides.

Jianmin Sun, Shin-ichiro Fujita and Masahiko Arai, Journal of Organometallic Chemistry 690 (2005) 3490 - 3497 and Wei-LI Dai, Sheng-Lian Luo, Shuang-Feng Yin and Chak-Tong Au, Applied Catalysis A: General 366 (2009) 2-12 describe the synthesis of compounds with a 2-oxo-[1,3] dioxolane group (cyclic carbonate) by transformation of carbon dioxide into cyclic carbonate.

For the production of 2-oxo-[1,3] dioxolane-4-carboxylic acid esters a process is desired which is easily performable and wherein the formation of by-products is avoided. Furthermore such process should be suitable to be performed continuously on an industrial scale and should have maximum yield and selectivity of 2-oxo-[1,3] dioxolane-4-carboxylic acid esters.

The intention of the present invention was therefore to find a process, in particular a continuous process, for the production of 2-oxo-[1,3] dioxolane-4-carboxylic acid esters, which is easily performable and fulfils the demands regarding yield and selectivity.
Accordingly we have now found the process claimed above.

Starting material is an oxirane-2-carboxylic acid ester of formula II 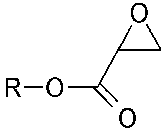 with R being an organic group comprising 1 to 10 carbon atoms. R may comprise of other atoms besides carbon and hydrogen, in particular oxygen or nitrogen, for example in form of hydroxyl groups, ether groups, nitro groups or amine groups.

Preferably R represents an organic group which contains only carbon and hydrogen atoms. Most preferably R is an alkyl group, in particular an alkyl group with 1 to 10 carbon atoms. Very particular reference is given to an alkyl group with 1 to 4 carbon atoms as a methyl, ethyl, n-propyl, iso-propyl or n-butyl group. In a most preferred embodiment of the invention, R is a methyl group.

Oxirane-2-carboxylic acid ester of formula II are reacted with carbon dioxide:

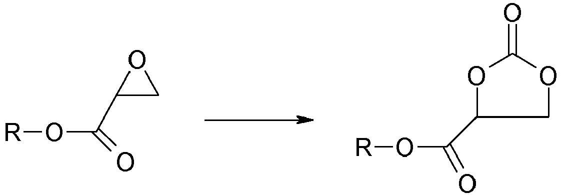

By addition of carbon dioxide the epoxy group is transferred to a cyclic carbonate group and the 2-oxo-[1,3] dioxolane-4-carboxylic acid ester of formula I is obtained.

Oxirane-2-carboxylic acid ester of formula II is reacted with carbon dioxide (CO₂) at a pressure of at least 35 bars and a temperature of 60 to 80°C.

As carbon dioxide is in the gaseous state under the conditions of the reaction, it is preferred to use carbon dioxide which has the desired pressure and obtain the desired pressure in the reactor. However, it would also be possible to combine carbon dioxide with other gases, in particular inert gases like nitrogen or helium.

More preferred is a pressure of at least 40 bars. The maximum pressure is preferably 100 bars, respectively 80 bars. In particular preferred is a pressure of 40 bars to 80 bars.

In a preferred embodiment the process of the invention is performed in presence of a catalyst. Suitable catalysts are salts, in particular halides. The cation of the halides may be an inorganic or organic cation. Suitable inorganic cations are metal cations from the first groups of the periodic system of elements, in particular the cations of sodium or potassium. Suitable organic cations are ammonium groups, in particular ammonium groups with four organic substituents to the nitrogen atom; such organic substituents being preferably aliphatic or aromatic groups that comprise no other atoms besides carbon, hydrogen, and oxygen atoms; most preferred are substituents which comprise no other atoms besides carbon and hydrogen, for example alkyl groups with 1 to 10 carbon atoms, a phenyl group or benzyl group or a phenyl, respectively benzyl group, which is further substituted by one to three alkyl groups. The halide may for example be fluoride, chloride, bromide or iodide, preferred is chloride and bromide, most preferred is bromide.

The catalyst is preferably used in amounts of at least 1.0 mol, more preferably of at least 1.5, most preferably of at least 2.0 mol per 100 mol of oxirane-2-carboxylic acid ester of formula II. However, there is no benefit and hence no need to use more than 10 mol, in particular more than 5 mol catalyst per 100 mol of oxirane-2-carboxylic acid ester of formula II.

The process may be performed in the presence of additional solvents. In a preferred embodiment no solvents are used.

The process may be performed in any kind of reactor, for example a tube or tank reactor.

In a preferred embodiment the process of the invention is a continuous process.

In such continuous process oxirane-2-carboxylic acid ester of formula II and carbon dioxide are fed continuously to the reactor, which is preferably a tube or more preferably a tank reactor and the obtained product is withdrawn continuously. The catalyst may be fed continuously to the reactor as well; however, it is also possible that the reactor may contain an heterogeneous catalyst either as a fixed bed or in suspension for example in form of an anion exchange resin loaded with halide.

The reaction time may be, for example, from half an hour to 24 hours.

The catalyst may be separated from the product obtained by usual means as filtration or extraction with a suitable solvent. In a preferred embodiment first the catalyst is separated from the product before other materials, in particular unreacted starting material, may be separated from the product.

Unreacted starting material may be separated from the product for example by distillation.

The obtained 2-Oxo-[1,3] dioxolane-4-carboxylic acid esters of formula I may be further purified by distillation; however it is one advantage of the invention that such a further purification of the product is not necessary and the product may be used in chemical processes, in particular for the synthesis of chemical compounds, or other technical applications without such a further purification.

The process of the invention is easily performable and 2-oxo-[1,3] dioxolane-4-carboxylic acid esters of formula I are obtained with high yield and selectivity. It is a particular advantage of the invention that the process can be easily performed continuously on industrial scale.

### Examples

### Synthesis of 2-oxo-[1,3]dioxolane-4-carboxylic acid methyl ester

The reaction was run in a 200 ml, steal, high pressure vessel fitted with overhead stirrer. To the vessel was added oxirane-2-carboxylic acid methyl ester (100g) and benzyl trimethylammonium bromide (5g, corresponding to 2.2 mol %) under ambient temperature and pressure. The vessel was then sealed and flushed three times with CO₂. The vessel was then pressurized with CO₂ to 5bar. The reaction mixture was then heated to 70°C and at which point the pressure of CO₂ was increased to 50bar. The reaction mixture was allowed to stir at the elevated temperature for 15h. The vessel was then allowed to cool to room temperature before degassing and flushing with N₂. The content of the vessel was then washed with water (3x100ml), extracting with dichloromethane (100ml). The organics were then dried with sodiumsulphate, filtered, and the solvent removed under vacuum, furnishing the product in 90.5% yield with a purity of greater than 95% without purification.

All examples listed in the Table below have been performed accordingly. Temperature, pressure and amount of catalyst have been modified as listed in the Table.

| Example | Catalyst Mol% | Pressure bar | Temperature °C | Reaction time hours | Yield Weight % | Colour of product |
|---|---|---|---|---|---|---|
| 1 (102) | 2.2 | 50 | 70 | 10 | 96.5 | yellow |
| 2 (112) | 2.2 | 45-50 | 70 | 10 | 98.8 | yellow |
| 3 (116) | 2.2 | 45-50 | 70 | 15 | 100 | Light yellow |
| 4 (139) | 1.1 | 45-50 | 70 | 15 | 91.5 | yellow |
| 5 (140) | 0.4 | 45-50 | 70 | 15 | 84.8 | yellow |
| 6 (142) | 2.2 | 40-50 | 70 | 15 | 94.9 | yellow |
| 7 (145) | 2.2 | 65-70 | 70 | 15 | 98.6 | yellow |
| C1 (142) | 2.2 | 25-30 | 70 | 15 | 85.7 | yellow |
| C2 (147) | 2.2 | 45-50 | 55 | 15 | 76.8 | Dark yellow |
| C3 (149) | 2.2 | 45-50 | 85 | 15 | 79.1 | Dark yellow |

## Claims

1. A process for the preparation of 2-oxo-[1,3] dioxolane-4-carboxylic acid esters of formula I 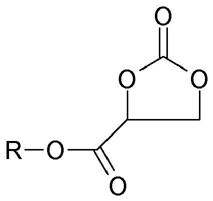 with R being an organic group comprising 1 to 10 carbon atoms, wherein
an oxirane-2-carboxylic acid ester of formula II 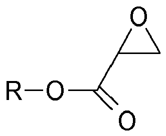 is reacted with carbon dioxide (CO₂) at a pressure of at least 35 bars and a temperature of 60 to 80°C.

2. The process according to claim 1, wherein R is a C1- to C10- alkyl group.

3. The process according to claim 1 or 2, wherein R is a methyl group.

4. The process according to any of claims 1 to 3, wherein the pressure is from 40 to 100 bars.

5. The process according to any of claims 1 to 4, wherein the process is performed in the presence of a catalyst selected from salts with a halide as an anion.

6. The process according to claims 5, wherein the catalyst is used in amounts of at least 1.0 mol catalyst per 100 mol oxirane-2-carboxylic acid ester of formula II.

7. The process according to any of claims 1 to 6, wherein the process is a continuous process.

## Patentansprüche

1. Verfahren zur Herstellung von 2-Oxo-[1,3]dioxolan-4-carbonsäureestern der Formel I 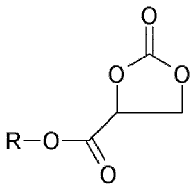 wobei R für eine organische Gruppe mit 1 bis 10 Kohlenstoffatomen steht, wobei
ein Oxiran-2-Carbonsäureester der Formel II 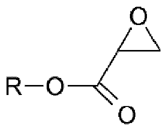 mit Kohlendioxid (CO₂) bei einem Druck von wenigstens 35 bar und einer Temperatur von 60 bis 80°C umgesetzt wird.

2. Verfahren nach Anspruch 1, wobei R für eine C1-bis C10-Alkylgruppe steht.

3. Verfahren nach Anspruch 1 oder 2, wobei R für eine Methylgruppe steht.

4. Verfahren nach einem der Ansprüche 1 bis 3, wobei der Druck 40 bis 100 bar beträgt.

5. Verfahren nach einem der Ansprüche 1 bis 4, wobei das Verfahren in Gegenwart eines aus Salzen mit einem Halogenid als Anion ausgewählten Katalysators erfolgt.

6. Verfahren nach Anspruch 5, wobei der Katalysator in Mengen von wenigstens 1,0 mol Katalysator pro 100 mol Oxiran-2-carbonsäureester der Formel II eingesetzt wird.

7. Verfahren nach einem der Ansprüche 1 bis 6, wobei es sich bei dem Verfahren um ein kontinuierliches Verfahren handelt.

## Revendications

1. Procédé de préparation d'esters d'acide 2-oxo-[1,3]dioxolane-4-carboxylique de formule I 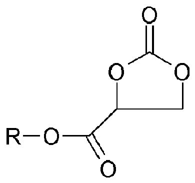 R étant un groupement organique comprenant de 1 à 10 atomes de carbone, **caractérisé en ce que**
un ester d'acide oxirane-2-carboxylique de formule II 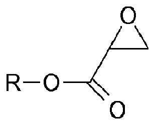 est réagi avec du dioxyde de carbone (CO₂) à une pression d'au moins 35 bars et une température allant de 60 à 80°C.

2. Procédé selon la revendication 1, **caractérisé en ce que** R est un groupement C₁ à C₁₀-alkyle.

3. Procédé selon la revendication 1 ou 2, **caractérisé en ce que** R est un groupement méthyle.

4. Procédé selon l'une quelconque des revendications 1 à 3, **caractérisé en ce que** la pression va de 40 à 100 bars.

5. Procédé selon l'une quelconque des revendications 1 à 4, **caractérisé en ce que** le procédé est réalisé en présence d'un catalyseur choisi parmi des sels ayant un halogénure comme anion.

6. Procédé selon la revendication 5, **caractérisé en ce que** le catalyseur est utilisé selon des quantités d'au moins 1,0 mol de catalyseur pour 100 mol d'ester d'acide oxirane-2-carboxylique de formule II.

7. Procédé selon l'une quelconque des revendications 1 à 6, **caractérisé en ce que** le procédé est un procédé continu.
